(19) Europäisches Patentamt
European Patent Office
Office européen des brevets

(11) **EP 3 254 628 B1**

(12) **EUROPEAN PATENT SPECIFICATION**

(45) Date of publication and mention
of the grant of the patent:
**11.12.2019 Bulletin 2019/50**

(51) Int Cl.:
***A61B 8/13*** *(2006.01)*

(21) Application number: **16746612.7**

(22) Date of filing: **02.02.2016**

(86) International application number:
**PCT/JP2016/053034**

(87) International publication number:
**WO 2016/125781 (11.08.2016 Gazette 2016/32)**

(54) **HANDHELD PROBE**

TRAGBARE SONDE

SONDE À MAIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.02.2015 JP 2015022248**

(43) Date of publication of application:
**13.12.2017 Bulletin 2017/50**

(73) Proprietor: **Canon Kabushiki Kaisha
Tokyo 146-8501 (JP)**

(72) Inventor: **ABE Hiroshi
Tokyo 146-8501 (JP)**

(74) Representative: **TBK
Bavariaring 4-6
80336 München (DE)**

(56) References cited:
**WO-A1-2012/090742       JP-A- 2013 084 923
JP-A- 2013 172 810       JP-A- 2013 208 422
JP-A- 2013 233 238**

EP 3 254 628 B1

## Description

Technical Field

The present invention relates to a handheld-type probe.

Background Art

PAI (Photoacoustic Imaging) is known as a technique for visualizing a light absorber inside an object (for example, a blood vessel in a living body). Photoacoustic imaging is a technique of creating image data of a distribution of a light absorber, utilizing the fact that, when an object is irradiated with light, a photoacoustic wave is generated from the light absorber because of the photoacoustic effect. Hemoglobin can be used as a light absorber, for example, whereby a blood vessel inside a living body can be visualized.

Meanwhile, ultrasonic imaging is known as a method of producing an image of structural information inside of an object. In ultrasonic imaging, ultrasound waves are transmitted to an object from a large number of detection elements (transducers) disposed in a probe. Image data is generated by receiving waves reflected from an acoustic impedance interface inside the object.

NPL 1 describes a technique of obtaining an image formed by photoacoustic imaging and an image formed by ultrasonic imaging with the same handheld-type probe.

Further prior art is disclosed in document WO 2012/090742 A1, describing a photoacoustic device and method for acquiring subject information, as well as in document JP 2013 233238 A, describing a photoacoustic measurement device and probe for the photoacoustic measurement device.

Citation List

Non-Patent Literature

NPL 1: J. J. Niederhauser, M. Jaeger, R. Lemor, P. Weber, and M. Frenz, "Combined ultrasound and optoacoustic system for real-time high-contrast vascular imaging in vivo", IEEE Trans. Med. Imaging 24, 436-440(2005)

SUMMARY OF INVENTION

Technical Problem

Generally, a photoacoustic probe has a circuit configuration dedicated to reception, and if it is to be utilized for ultrasonic imaging, an ultrasound wave transmission circuit needs to be added. However, this causes an increase in production cost. Moreover, the switching circuit for switching between transmission and reception can generate noise. Furthermore, the transmission characteristics given to the detection elements may reduce the reception characteristics.

The present invention was made in view of the problems described above, and it is an object of the invention to provide a handheld-type probe that is suitable for photoacoustic imaging and ultrasonic imaging and has a simple structure.

Solution to Problem

To achieve the above objective, one aspect of the present invention provides a handheld-type probe according to claim 1. Another aspect of the present invention provides an object information acquiring apparatus according to claim 14. Further advantageous developments of the present invention are defined in the dependent claims.

Advantageous Effects of Invention

The present invention can provide a handheld-type probe that is suitable for photoacoustic imaging and ultrasonic imaging and has a simple structure.

Further features of the present invention will become apparent from the following description of exemplary embodiments with reference to the attached drawings.

Brief Description of Drawings

Fig. 1 is a diagram showing a configuration example of a handheld-type probe of Embodiment 1.
Fig. 2 is a cross-sectional view of the handheld-type probe of Embodiment 1.
Figs. 3A to 3C are diagrams showing a process flow of Embodiment 1.
Fig. 4 is a diagram showing how a received signal looks in Embodiment 1.
Fig. 5 is a diagram showing a configuration example of a cover member of Embodiment 2.
Figs. 6A and 6B are diagrams showing a handheld-type probe of Embodiment 3.
Fig. 7 is a block diagram showing a configuration of an object information acquiring apparatus.

DESCRIPTION OF EMBODIMENTS

Preferred embodiments of the present invention will be hereinafter described with reference to the drawings. Note, the sizes, materials, shapes, and relative arrangements or the like of constituent components described below should be changed as required in accordance with the configurations and various conditions of the apparatus to which the invention is applied. Therefore, the description is not intended to limit the scope of this invention to the specifics given below.

The present invention relates to a technique of detecting an acoustic wave that propagates from an object, and generating and acquiring characteristic information relating to the inside of the object. The present invention is therefore understood as an object information acquiring apparatus and a control method thereof, or an object information acquiring method and a signal processing method. The present invention is also understood as a program for executing these methods on an information processing apparatus that has hardware resources such as CPUs, and a memory medium that stores this program.

The object information acquiring apparatus of the present invention includes apparatuses that utilize photo-coustic tomography techniques, whereby light (electromagnetic wave) is projected to an object, and an acoustic wave that was generated at a specific position inside or on a surface of the object and propagated therefrom according to the photoacoustic effect is received (detected). Such an object information acquiring apparatus can also be referred to as a photoacoustic imaging apparatus, because it acquires characteristic information relating to the inside of the object in the form of image data or the like based on photoacoustic measurements.

The object information acquiring apparatus of the present invention includes apparatuses that utilize ultrasound echo techniques, whereby an acoustic wave is transmitted to an object, and a wave reflected from inside of the object (echo wave) is received to acquire object information as image data.

The characteristic information in a photoacoustic apparatus includes source distributions of acoustic waves generated by light irradiation, initial sound pressure distributions inside the object, or, optical energy absorption density distributions, absorption coefficient distributions, and density distributions of tissue-forming substances, which are deduced from the initial sound pressure distributions. More specifically, these may be a distribution of oxyhemoglobin/deoxyhemoglobin concentration, a blood component distribution such as an oxygen saturation distribution obtained therefrom, or fat, collagen, or water distributions. The characteristic information may be obtained as information relating to a distribution of positions inside the object rather than numerical data. Namely, the object information may be distribution information such as an absorption coefficient distribution and an oxygen saturation distribution. Characteristic information originating from photoacoustic waves can also be referred to as functional information that indicates differences in function caused by a substance inside the object.

The characteristic information acquired with an ultrasound echo apparatus is information that reflects differences in acoustic impedance of tissues inside the object. The characteristic information originating from ultrasound echoes can also be referred to as morphological information that reflects the structure inside the object.

Acoustic waves referred to in the present invention are typically ultrasound waves, including elastic waves that are called sound waves or acoustic waves. An acoustic wave generated by the photoacoustic effect is referred to as a photoacoustic wave, or a light-induced ultrasound wave. An electrical signal converted from an acoustic wave by a probe or the like is referred to as an acoustic signal.

<Embodiment 1>

Embodiment 1 of the present invention will now be described with reference to the drawings. In the following description, unless otherwise specified, "photoacoustic wave" shall refer to a photoacoustic wave generated from a light absorber inside or on a surface of an object. On the other hand, a photoacoustic wave generated from a light absorber disposed on a probe shall be referred to as "transmitted ultrasound". When they need to be clearly distinguished from each other, they will be referred to as "object-originated photoacoustic wave " and "probe-originated photoacoustic wave", respectively. An echo wave that is a reflection of transmitted ultrasound from the surface or inside of the object shall be referred to as "ultrasound echo". These names are for easy distinction and not intended for limiting respective elastic waves to specific wavelengths.

<Configuration of the probe>

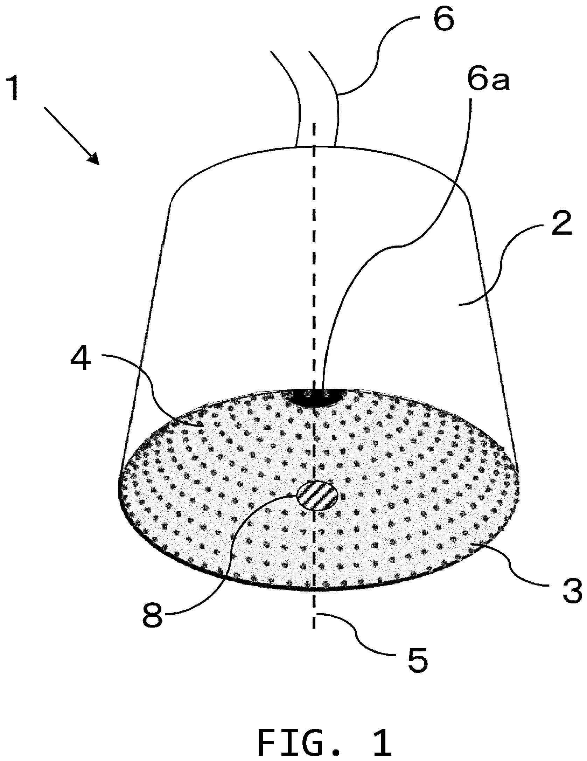

Fig. 1 is a diagram showing a configuration example of a handheld-type probe of this embodiment. The handheld-type probe 1 has a cylindrical grip portion 2 for an operator to hold. The grip portion 2 also functions as a housing for holding various constituent elements. The grip portion 2 need not necessarily be cylindrical. It may have an elliptic cylindrical or prismatic shape, and provided with protrusions and recesses for allowing easier grip by the operator.

A detection surface 3 is a surface intersecting a center axis 5 of the grip portion 2. A plurality of detection elements 4 (transducers) are provided on the detection surface 3. The handheld-type probe 1 of Fig. 1 can project light introduced from a light source (not shown) by way of an optical system 6 onto an object from an irradiation end 6a that is disposed substantially at the center of the detection surface 3.

In Fig. 1, the detection surface 3 is a curved surface of a shape that is concave relative to the object. The detection surface 3 being a curved surface can provide an effect that the measurement target of ultrasonic imaging can be focused in a region of interest inside the object. An ellipsoid partial segment is a preferable shape for the curved surface, and a spherical cap is even more preferable. However, the detection surface 3 may also be a flat surface. The handheld-type probe 1 is pressed against the object such that the detection surface 3 faces the object.

The plurality of detection elements 4 receive photoacoustic waves and ultrasound echoes that propagate from the object, convert the waves to electrical signals and output the signals. The arrangement of the plurality of detection elements 4 is not limited to a specific pattern. They can be arranged in square grids, for example, or various other patterns can be used, such as a rectangle, polygons with 5 or more sides, circle, ellipse, fan-shape, concentric circles, etc. The detection elements 4 may be arranged in concentric rings. The elements will then be arranged with higher symmetry so that a favorable reconstructed image can be obtained.
The elements in this case should preferably be arranged in concentric rings around the irradiation end of light.

For the detection elements 4, piezoelectric elements, cMUT (Capacitive Micromachined Ultrasonic Transducers), PVDF (PolyVinylidene DiFluoride), Fabry-Perot sensors and the like may be used. The size and alignment intervals of the elements may be determined from the viewpoints of reception sensitivity and directionality in accordance with the purpose of use.

Preferably, 70% or more of the detection surface 3 should be covered by the plurality of detection elements 4 arranged on the detection surface 3, and more preferably, 85% or more of the detection surface 3 should be covered. This way, the photoacoustic waves and ultrasound echoes propagating from the object can be efficiently received from various angles so that high-precision image data can be generated.

Each of the detection elements 4 should desirably have an equal solid angle relative to the region of interest. For this reason, the detection elements 4 should desirably arranged in a homoscedastic pattern such as a spiral or Fibonacci pattern. It is desirable, in order to increase the number of detection elements 4 for higher SNR, that the detection elements 4 be arranged in concentric rings relative to the irradiation end 6a.

For the light source (not shown), a pulsed laser apparatus is preferable. Light of a wavelength that matches the absorption characteristics of the light absorber inside the object is preferable as the laser light. For hemoglobin in blood, for example, near infrared light is preferable. Detailed information such as oxygen saturation can be obtained with the use of a wavelength tunable laser that generates light of a plurality of wavelengths. Flash lamps and LEDs can also be used instead of a laser apparatus.

Various optical components can be used for the optical system 6 for propagating the light from the light source, such as fiber bundles, mirrors, prisms, waveguides, and the like. The propagated light is projected to the object from the irradiation end 6a, which may be a distal end of a fiber bundle.

As will be described in detail later, a cover member 7 is provided on one side of the handheld-type probe 1 that is to be contacted to the object. The light absorber 8 in the drawing is arranged on the cover member 7. Namely, the cover member functions as a light-absorber supporting member in this embodiment. When the detection surface 3 of the handheld-type probe 1 is pressed against the object such as to face it, the cover member is positioned between the object and the detection surface.

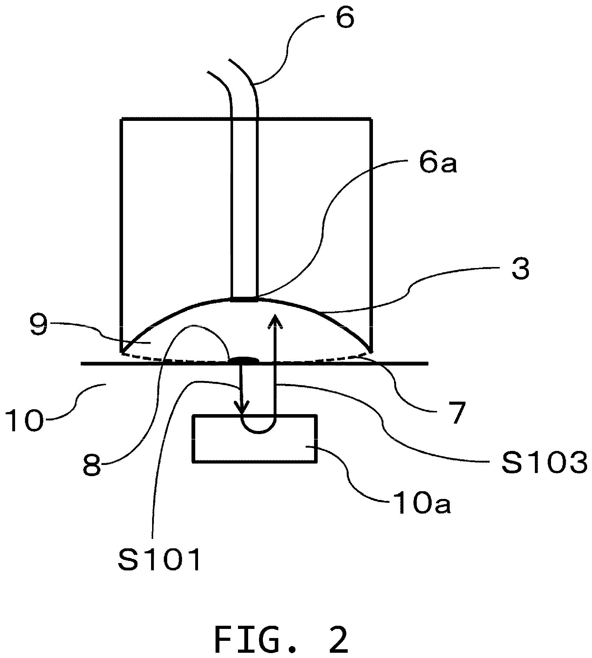

Fig. 2 is a cross-sectional view of the handheld-type probe 1 in contact with an object. Since the detection surface 3 is a concave surface in this embodiment, when the housing is pressed against the object 10, there is formed a gap. The cover member 7 is a component that makes contact with the object 10 at this time. In other words, a sealed space is formed between the cover member 7 and the detection surface 3 provided with the detection elements 4.

This sealed space is filled with a coupling medium 9 for acoustic matching between the detection elements 4 and the object 10. Suitable coupling medium 9 would have an acoustic impedance close to that of the object 10. Water or castor oil can favorably be used as the coupling medium 9, for example. Coupling medium 9 that has high transmissivity of the irradiated light is used in order to propagate the light to the object 10.

The cover member 7 should preferably have flexibility so as not to impede propagation of photoacoustic waves and ultrasound echoes. A soft membrane such as rubber is preferable, for example. To propagate the irradiated light to the object 10, a material having light transmissivity is used. By filling the cover member 7 with the coupling medium 9, acoustic matching between the detection elements and the object is achieved. The cover member 7 also prevents direct contact between the object 10 and the coupling medium 9. In Fig. 2, when the cover member 7 filled with the coupling medium is pressed against the object 10, the cover member 7 deforms in conformity to the shape of the object 10 and makes tight contact with the object 10.

The coupling medium may be applied in addition between the object 10 and the cover member 7. This will increase the sealing and acoustic matching properties between the object 10 and the cover member 7. Also, the movement of the probe 1 during measurement relative to the object 10 will be made more smoothly. The cover member 7 may be a bag-shaped component and configured to be removably attached to the probe 1. Bag-shaped cover member 7 already filled with a coupling medium may be replaced for each measurement or object for example, which is effective in hygienic aspects.

The cover member filled with the coupling medium should preferably be provided also when the detection surface 3 is flat, from the viewpoints of protection of detection elements, improved acoustic impedance matching properties, and better comfort of the person being inspected.

<Light absorber>

The cover member 7 has a dot-like light absorber 8. When part of the light from the irradiation end 6a is projected to the light absorber 8, transmitted ultrasound S101 is generated by the photoacoustic effect. This transmitted ultrasound reaches the object 10, is reflected at a region of interest 10a or the like, and produces an ultrasound echo S103. This is received by the detection elements, whereby ultrasonic imaging is accomplished. On the other hand, the rest of the projected light reaches the object, and causes a photoacoustic wave to be generated. This is received by the detection elements, whereby photoacoustic imaging is accomplished. This way, the ultrasonic imaging and photoacoustic imaging can be performed with a single, simple-structured probe that does not have a function of transmitting ultrasound waves.

From the viewpoint of efficient generation of transmitted ultrasound, the light absorber 8 should preferably be disposed on a center axis 5 (optical axis) where the light intensity is high. The dot size of the light absorber 8 affects the frequency of the transmitted ultrasound. Therefore, the dot size of the absorber is preferably determined such that the frequency of the generated transmitted ultrasound falls within the range of the band sensitivity of the detection elements 4. One example of the dot size of the light absorber 8 is about 400 $\mu$m in diameter when the thickness of the cover member 7 is 500 $\mu$m. This way, irregularities relative to the object surface can be reduced.

The light absorber 8 may be made of a material that can efficiently generate transmitted ultrasound such as, for example, a material that contains carbon. The light absorber 8 absorbing the light completely will affect the photoacoustic imaging. Therefore, the size and material of the light absorber 8 are determined such that part of light will avoid or transmit the light absorber 8 to reach the object 10. As one example, one case is considered wherein the light-irradiated region is 15 mm in diameter in a handheld-type probe with an about 500 mm diameter detection surface 3. Most of the irradiated light can reach the object if the light absorber 8 of about 400 $\mu$m diameter as described above is used in this probe, so that the photoacoustic imaging will be hardly affected.

When the position of the light absorber 8 is largely displaced from the center axis 5 by the contact with the object, the signal intensity of the transmitted ultrasound is lowered. It will therefore be advantageous to use a hard material when making the cover member 7 partly for a region in the vicinity of the grip portion 2 only. To deal with such displacement, a configuration could also be considered wherein a plurality of dots are arranged on the cover member so that at least one of the dots will be present on the optical axis. With a plurality of dots, however, it will be harder to determine which of the dots the obtained ultrasound echo originates from. Therefore, it is more preferable to dispose only one dot.

When the cover member 7 is designed replaceable as mentioned above, a plurality of cover members having different materials, sizes, numbers, positions and the like of the light absorber may be prepared and replaced in accordance with the measurement contents. The cover member 7 may also be configured to include a valve-like injection port. Thereby, the coupling medium can be filled between the cover member 7 and the detection surface through the injection port only at the time of measurement. As a result, leakage of coupling medium caused by breakage of the cover member 7 during storage or transportation can be prevented. The injection port should preferably be provided in a peripheral edge portion of the detection surface so that it does not hinder measurements.

<Process flow>

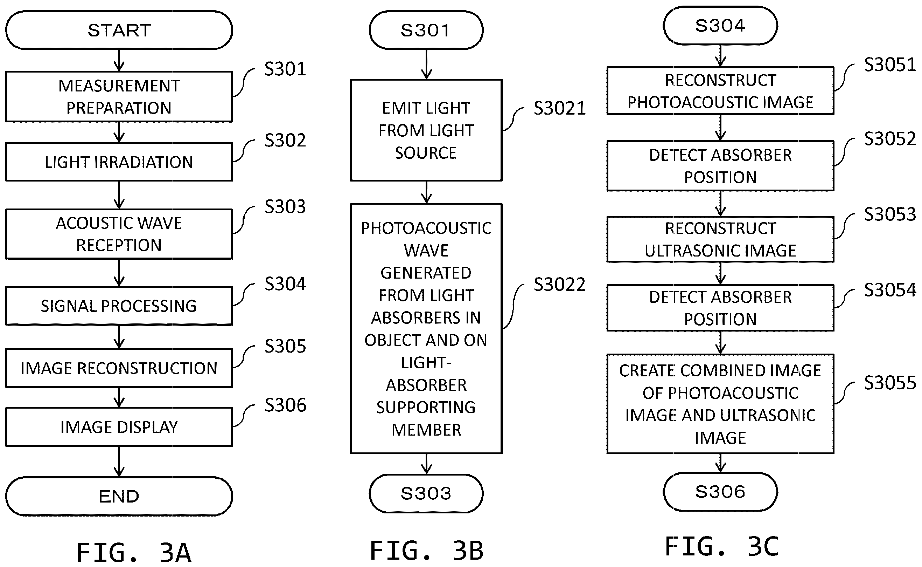

Next, a measurement sequence according to this embodiment will be described with reference to Fig. 3. Fig. 3A shows a general process flow. Fig. 3B provides a detailed explanation of step S302, and Fig. 3C provides a detailed explanation of step S305.

(Step S301: Measurement preparation)

In this step, preparations are made, such as starting up the apparatus, attaching the cover member 7 to the handheld-type probe 1, applying ultrasound gel to the probe, and bringing the probe into contact with the object. This brings about a condition where measurements are possible as shown in the schematic diagram of Fig. 2. In this example, a region of interest (ROI) 10a inside the object 10 is the measurement target.

(Step S302: Light irradiation)

At step S3021, light that has been emitted from the light source and transmitted by the optical system 6 is projected from the irradiation end 6a. At step S3022, the light absorber disposed in the cover member 7 absorbs the light, whereby a probe-originated photoacoustic wave (transmitted ultrasound) S101 is generated. Part of the transmitted ultrasound is received by the detection elements 4 via the coupling medium 9. Another part of the transmitted ultrasound is reflected at a structural body inside the region of interest 10a or an acoustic impedance interface such as the object surface and becomes the ultrasound echo S103, which is received by the detection elements 4. The light irradiation process may be performed repeatedly a number of times.
As a light absorber in the region of interest 10a absorbs light, an object-oriented photoacoustic wave S102 is generated. The photoacoustic wave is received by the detection elements 4 via a region inside the object and the coupling medium 9.

(Step S303: Acoustic wave reception)

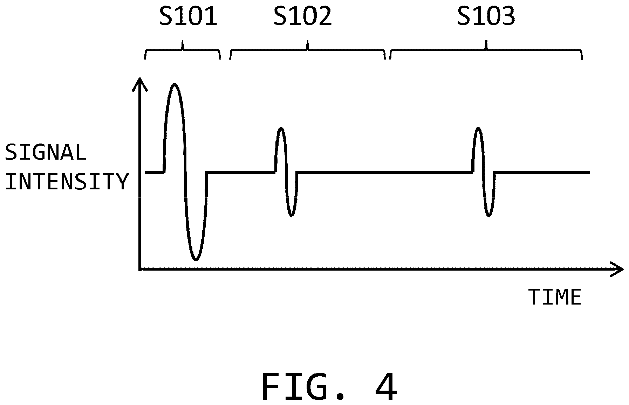

Fig. 4 shows a typical example of an electrical signal based on an acoustic wave detected by the detection elements 4. The horizontal axis represents the time that has passed after the light irradiation. The electrical signal is determined by the length of the path the acoustic wave has passed after being generated and the speed of sound of the region the acoustic wave has passed. The vertical axis represents the intensity of the electrical signal.
Referring to Fig. 4, first, a high-intensity signal is observed. This corresponds to the transmitted ultrasound S101, which originates from the light absorber 8 disposed inside the cover member. The signal observed next corresponds to the photoacoustic wave S102 generated from the light absorber inside the region of interest. The signal observed next corresponds to the ultrasound echo S103 that is the transmitted ultrasound S101 reflected by a structural body inside the region of interest.
To be precise, the photoacoustic wave S102 generated from the region of interest is also reflected by the structural body. However, the energy generated by this reflected wave is low, and so is the signal intensity, and it is generally negligible or unobservable. When the size of the region of interest is small relative to the distance from the dot-like light absorber disposed inside the cover member, both signals are not observed at the same time.

(Step S304: Signal processing)

In this step, signal processing is performed as required to the electrical signal output from each detection element. Examples include an amplification process, a digitization process on an analog electrical signal, a gain process in accordance with the path length or amount of light of the acoustic wave, and a process for correcting element characteristics.

(Step S305: Image reconstruction)

In this step, a photoacoustic imaging process that uses the photoacoustic wave and an ultrasonic imaging process that uses the ultrasound echo are performed. Various known techniques can be used in image reconstruction at steps S3051 and S3053. The processing in this step is not limited to the flow shown in Fig. 3C, and an ultrasonic image and a photoacoustic image can be generated in parallel steps.
For the reconstruction of photoacoustic waves, a back projection algorithm in 3D space can be applied. For example, the initial sound pressure distribution $p_0$ (r) can be estimated temporospatially by the following equation (1) by a UBP (Universal Back-Projection) method.
[Math. 1]

$$p_0(r) = \int_{\Omega_0} b(r_0, t = |r - r_0|) \frac{d\Omega_0}{\Omega_0} \qquad \cdots (1)$$

Term b ($r_0$, t) corresponding to projection data in this case is expressed by equation (2).
[Math. 2]

$$b(r_0,t) = p(r_0,t) - 2t\frac{\partial p(r_0,t)}{\partial t} \qquad \cdots(2)$$

Term $d\Omega_0$ that represents the solid angle of a receptor relative to a given data region is expressed by equation (3).
[Math. 3]

$$d\Omega_0 = \frac{dS_0}{|r - r_0|^2}\cos\theta \qquad \cdots(3)$$

Here, p(r) represents the photoacoustic wave signal acquired at S100, r represents the position of each detection element, t represents time, and $\theta$ represents the angle made by the receptor and a given data region.
When the distance between the sound source and the measurement position is large enough in comparison to the size of the sound source, the projection data term b ($r_0$, t) may be expressed by equation (4).
[Math. 4]

$$b(r_0,t) = -2t\frac{\partial p(r_0,t)}{\partial t} \qquad \cdots(4)$$

The cover member 7 deforms when contacted to the object, so that the position of the light absorber 8 provided at the cover member 7 may have changed from the position when the cover member is not in contact with the object. Therefore, correction may be made in consideration of the influence of this displacement of the light absorber 8 on the signal. Position information relating to the light absorber can be acquired at step S3052 based on the photoacoustic wave S101 directly incident from the light absorber 8 to the detection elements. At step S3053, a back projection algorithm was applied in 3D space similarly to the case with the photoacoustic image when calculating an image reconstruction I(r) of the reflected ultrasound (Equation (5)).
[Math. 5]

$$I(r) = \frac{1}{M}\sum_{i=1}^{M}\omega * S_i(t+\tau) \qquad \cdots(5)$$

Here, $\tau$ represents a delay time that is determined by dividing the sum of the distance from the light absorber inside the membrane to a projection position voxel and the distance from an i-th detection element to the projection position voxel by the speed of acoustic propagation.
$\omega$ represents a weight factor such as a window function and is changed in accordance with the directionality of the detection elements, and desired resolution or SN of the reconstructed image.
Projection data that takes account of a displacement inside the object can be visualized by extracting sampling data Si from time-series signals received by each detection element with the same phase relative to the projection position voxel, and adding the sampling data.

(Step S306: Image display)

Ultrasonic image data and photoacoustic image data generated by image reconstruction are displayed on a display device such as a liquid crystal display. Any displaying techniques can be used, such as superposing both images, displaying them side by side, or displaying them alternately. Note, the effects of the present invention are achieved even when the images are not displayed but stored as image data. Further, the effects of the present invention are achieved with RAW data output from the detection elements, or electrical signals subjected to a correction process only prior to image reconstruction, since these data contain the components originating from the light absorber of the cover member.

As described above, with this embodiment, the ultrasonic imaging and photoacoustic imaging can be performed with a single, simple-structured probe provided only with the detection elements that do not have a function of transmitting ultrasound waves. Therefore, a cost reduction, an improved availability factor, facilitation of maintenance and the like can be expected for a diagnosis that uses a plurality of modalities.

<Variation Example>

When a material having flexibility is used for the cover member 7, the position of the light absorber on the cover will change in accordance with the strength with which the probe is pressed against the object and the direction. As a result, the positional relationship between the light absorber that is the sound source and the object changes, which changes the arrival time of the transmitted ultrasound from the sound source to the object, and arrival time of the ultrasound echo to the detection elements. It is then possible that the position where a component originating from the reflected wave from the region of interest is contained in the digitized electrical signal is displaced, which then affects the reconstruction accuracy.

The longer the distance between the sound source and the region of interest, the higher the degree of attenuation of the transmitted ultrasound, so that the SN ratio of the reconstructed image may be affected. Moreover, as the irradiated light is attenuated and the distance between the irradiation end of the light and the light absorber becomes longer, the intensity of the transmitted ultrasound is reduced, so that the SN ratio of the reconstructed image may be affected.

If the change in position of the light absorber due to a deformation of the cover member is known, the position in the digital signal of the signal component originating from the reflected wave that propagated from the region of interest can be determined, and reconstruction can be performed correctly. Corrections such as gain processing can also be performed to deal with attenuation of the transmitted acoustic wave itself, and reduced intensity of the transmitted ultrasound in accordance with the attenuation of irradiated light. A method will be explained in this variation example whereby the position of the light absorber is determined in advance, in a state wherein the probe is pressed against the object (abutted state).

First, the user carries out preliminary light irradiation in the abutted state. This causes not only the light absorber in the cover member but also the light absorber inside the object to generate a photoacoustic wave, but they can be distinguished since their detection times and intensities differ. Image reconstruction is performed by using the electrical signal originating from the light absorber in the cover member. Consequently, the position information relating to the light absorber can be obtained.

According to this variation example, the position of the light absorber disposed in the cover member is determined, so that correct image reconstruction is realized. In this variation example, light irradiation for obtaining position information and light irradiation for reconstructing an image need not necessarily be performed separately. Namely, after obtaining an electrical signal such as the one shown in Fig. 4, the position of the light absorber may be determined by image reconstruction with the use of the part S101 first, and then part S102 (corresponding to the object-originated photoacoustic wave) and part S103 (corresponding to the ultrasound echo) may be used for the image reconstruction.

As another variation example, a configuration wherein the irradiation end of the light is not included in the housing of the grip portion can be considered. In this case, an optical component other than the probe for emitting light will be necessary. On the other hand, the degree of freedom in the position relationship between the light irradiation position and the light absorber will increase.

As a further variation example, a configuration wherein the cover member is a bag-shaped component can be considered. When a matching member is to be sealed inside a region defined by the detection surface and the cover membrane, the detection elements 4 may need to be provided with water-proof treatment. However, if the cover member is a bag-shaped component, the necessity to provide the detection elements 4 with water-proof treatment can be decreased. In this case, too, the bag-shaped cover member can be configured to be removably attached to the grip portion. An injection port for introducing the matching member can be provided also in the case with a bag-shaped cover member.

<Embodiment 2>

In Embodiment 1, only one dot of light absorber is provided on the cover member. In this case, when there is a region inside the object, which the transmitted ultrasound generated from the light absorber can hardly reach, the accuracy of image reconstruction of this region will be reduced. Such reduction can also occur when the position of the light absorber is distanced from the region of interest, and when the transmitted ultrasound has a frequency that can easily be attenuated. In this embodiment, therefore, a plurality of dots of light absorbers having mutually different absorption wavelength peaks are disposed on the cover member 7.

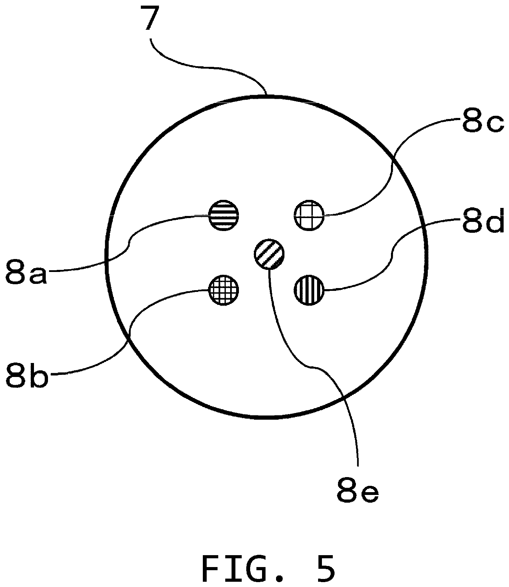

Fig. 5 shows how the cover member 7 of this embodiment looks when viewed from the object side. On the cover member 7, light absorber 1 (8a), light absorber 2 (8b), light absorber 3 (8c), light absorber 4 (8d), and light absorber 5 (8e) that have different light absorption characteristics are disposed. The positions where the respective light absorbers are disposed are preferably within a range that receives the light from the irradiation end. In Fig. 5, the irradiation end and detection elements that are present on the opposite side of the cover member are not shown.

In this embodiment, a wavelength tunable laser apparatus is used as the light source. Light of a wavelength suited to each light absorber is emitted per pulse or per time unit so that transmitted ultrasound (probe-originated photoacoustic wave) is generated, to detect an ultrasound echo. As a result, five types of reception signals having mutually different sound source positions can be obtained. The acoustic wave signals stored in a memory may then be averaged, or the reconstructed images each generated from the acoustic signals may be averaged, whereby a high SN ratio image can be obtained.

The object-originated photoacoustic signals contained in the five types of reception signals described above each result from irradiated light of different wavelengths and the types of the signals are distinguishable from the spectral information. High-precision photoacoustic imaging can be performed by using these signals. An oxygen saturation distribution image can be obtained highly accurately for example, if the five types of light include light of wavelengths easily absorbed by oxyhemoglobin and deoxyhemoglobin, respectively.

With this embodiment, photoacoustic imaging and ultrasonic imaging of a wide region inside an object can be realized with a single and simple-structured probe. While five types of light absorbers indicated by reference numerals 8a to 8e are provided in Fig. 5, the number and arrangement are not limited to this.

<Embodiment 3>

In the embodiments described above, a matching member is sealed inside the cover member 7. For the probe of this embodiment, a gel member 11 is used, which is a medium that has an acoustic impedance similar to that of a soft living tissue and transmits irradiated light. Thereby, the reflection and loss of the acoustic wave between the detection elements and the object can be reduced.

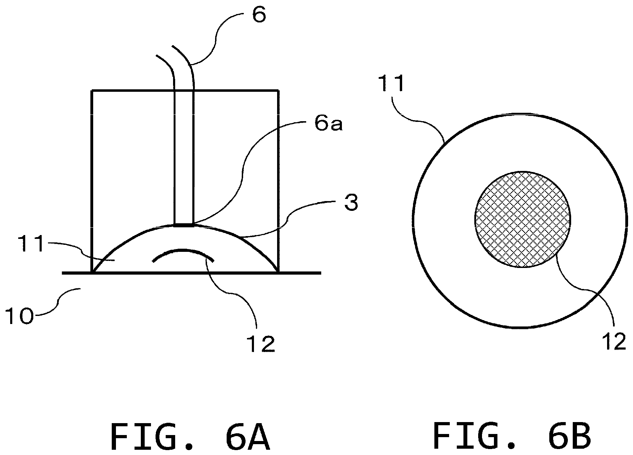

Fig. 6A is a cross-sectional view showing the structure of the handheld-type probe 1 according to this embodiment. One side of the gel member 11 has a shape conforming to the curved surface of the detection surface 3 so that air will not be entrapped. The side that makes contact with the object 10 has a shape suited to the object surface. The gel member may be designed to be detachable so that it can be replaced for each object, which is hygienic. Further, a sheet-like light absorber 12 having a radius of curvature substantially equal to that of the detection surface 3 is disposed inside this gel member 11 on the center axis 5 of the optical path. Namely, the gel member functions as a light-absorber supporting member in this embodiment.

Fig. 6B shows how the gel member 11 looks when viewed from the object side. The sheet-like light absorber 12 is disposed such as to cover the optical axis so as to cause the transmitted ultrasound to be generated efficiently. The material, thickness, size and the like of the sheet-like light absorber 12 are designed such as not to absorb all the irradiated light but such as to allow part of the light to be transmitted to the object. Acoustic transmissivity is also required of the sheet-like light absorber. For the material of the sheet-like light absorber, a gold, silver, or aluminum thin film may be used for example.

Of the photoacoustic waves generated by the sheet-like light absorber 12, those that propagate to the opposite side of the detection elements (transmitted ultrasound) travel to the center of curvature of the sheet-like light absorber 12. Therefore, if the center of curvature of the detection surface 3 coincides with the center of curvature of the sheet-like light absorber 12, the transmitted ultrasound propagates toward this center of curvature in a converged manner. A large signal amplification is formed at the center of radius as a result, so that high-precision image data is obtained. Artifacts are minimized since the reception focus of the photoacoustic wave is at the center of curvature, so that the image has a high SN ratio. Consequently, the effect of favorably obtaining an image of the inside of the region of interest is achieved.

When the gel member 10 is designed replaceable as mentioned above, a plurality of gel members having different materials, sizes, numbers, positions and the like of the sheet-like light absorber may be prepared and replaced in accordance with the measurement contents.

The thicker the light absorber, the lower the frequency of the generated photoacoustic wave, for example. In this case, the photoacoustic wave is hardly attenuated and reaches deep into the object, so that a deep portion can be visualized. On the other hand, the thinner the light absorber, the higher the frequency of the photoacoustic wave. In this case, attenuation can readily occur, but the measurement resolution will be higher. Thus gel members can be replaced in accordance with the depth or size of the measurement target to change the frequency of the photoacoustic wave.

According to this embodiment, a very fine ultrasonic image and photoacoustic image of an object can be obtained with the use of a single and simple-structured probe.

<Variation Example>

While a sheet-like light absorber has been described here, a dot-like or spherical light absorber may be embedded inside or near the surface of the gel member. In this case, too, ultrasonic imaging wherein a photoacoustic wave is used as the transmitted ultrasound can be realized with a simple structure. A plurality of light absorbers having mutually different light absorption characteristics may be embedded.

<Embodiment 4>

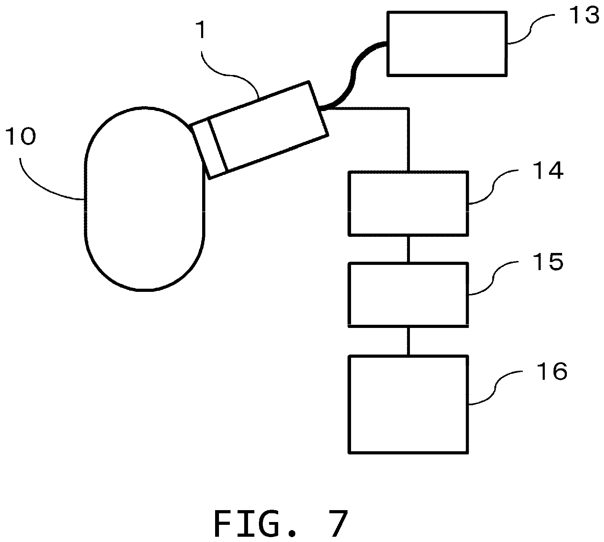

In this embodiment, an object information acquiring apparatus that uses the handheld-type probe 1 shown in various embodiments above will be described with reference to Fig. 7. This apparatus can generate image data of the inside of an object with two modalities, i.e., ultrasonic imaging and photoacoustic imaging. The process flow of Fig. 3 is executed in actuality in an object information acquiring apparatus such as the one in this embodiment.

The measurement target of the apparatus is the object 10. The apparatus includes the probe 1, a light source 13, a signal processing unit 14, an information processing unit 15, and a display unit 16. However, the apparatus does not necessarily require the display unit 16, and may be configured such that the reconstructed image data is stored in a memory.

The above-described ones of the probe 1 and light source 13 are used. The signal processing unit 14 performs the process of step S304 in Fig. 3. For the signal processing unit 14, an amplifier, an AD converter, and various correction circuits can be used. For the information processing unit 15, an information processing apparatus that includes a CPU, a memory and the like and operates in accordance with a program or the like, such as a PC or work station is suitable. For the display unit 16, any image displaying apparatus such as a liquid crystal display, or an organic EL display, can be used.

<Other Embodiments>

The present invention can also be embodied with a computer (or devices such as CPUs and MPUs) of a system or apparatus that realizes the functions of the embodiments described above by reading and executing a program recorded in a memory apparatus. Also, for example, the present invention can be embodied by a method including the steps that are executed by a computer of a system or apparatus that realizes the functions of the embodiments described above by reading and executing a program recorded in a memory apparatus. For this purpose, the program mentioned above is provided to the computer, for example via a network, or from various types of recording media that can constitute the memory apparatus mentioned above (i.e., computer-readable memory media that hold data non-temporarily). Therefore, the computers (including devices such as CPUs and MPUs), methods, programs (including program codes and program products), and computer-readable recording media that hold the programs non-temporarily mentioned above are all included in the scope of the present invention.

While the present invention has been described above with reference to typical embodiments, it should be understood that the present invention is limited to the scope of the appended claims.

**Claims**

1. A handheld-type probe (1), comprising:

   a grip portion (2);
   a plurality of detection elements (4) configured to receive an acoustic wave and output an electrical signal;
   a detection surface (3) where the plurality of detection elements (4) are disposed; and
   a light-absorber supporting member (7) where a light absorber (8) is disposed, the light absorber (8) absorbing light emitted from a light source (13) and generating an acoustic wave, wherein
   when the handheld-type probe (1) is pressed against an object (10) such that the detection surface (3) faces the object (10), the light-absorber supporting member (7) is positioned between the object (10) and the detection surface (3) in contact with the object, wherein
   the detection surface (3) is configured to have a shape that is concave relative to the object (10).

2. The handheld-type probe (1) according to claim 1, further comprising an irradiation end (6a) disposed on the detection surface (3) and emitting the light.

3. The handheld-type probe (1) according to any one of claim 1 or 2, wherein

the light-absorber supporting member (7) is a membrane; and
the light-absorber is provided at the membrane.

4. The handheld-type probe (1) according to claim 3, further comprising a coupling medium (9) filled between the light-absorber supporting member (7) and the detection surface (3).

5. The handheld-type probe (1) according to any one of claim 1 or 2, wherein
the light-absorber supporting member (7) is a bag-shaped member; and
the light-absorber is provided at the bag.

6. The handheld-type probe (1) according to claim 5, further comprising a coupling medium (9) filled in the bag-shaped member.

7. The handheld-type probe (1) according to any one of claims 3 to 6, wherein the light absorber (8) is dot-like.

8. The handheld-type probe (1) according to any one of claims 3 to 7, comprising a plurality of light absorbers configured to have mutually different light absorption characteristics.

9. The handheld-type probe (1) according to claim 2, wherein
the light-absorber supporting member (7) is a membrane, and
the light absorber (8) is provided at the membrane on an optical axis (5) that extends from the irradiation end (6a).

10. The handheld-type probe (1) according to any one of claims 1 or 2, wherein
the light-absorber supporting member (7) is a gel, and
the light-absorber is provided inside the gel.

11. The handheld-type probe (1) according to claim 10, wherein the light absorber (8) is sheet-like.

12. The handheld-type probe (1) according to claim 11, wherein the sheet-like light absorber (8) is configured to have a radius of curvature substantially equal to that of the detection surface (3).

13. The handheld-type probe (1) according to any one of claims 1 to 12, wherein the light-absorber supporting member (7) is removably attached to the grip portion (2).

14. An object information acquiring apparatus, comprising:

the handheld-type probe (1) according to any one of claims 1 to 13; and
an information processing unit (15), wherein
the information processing unit (15) is configured to acquire characteristic information relating to the inside of an object (10) by using an electrical signal originating from an acoustic wave that has propagated from the object (10), with the handheld-type probe (1) being pressed against the object (10) such that the detection surface (3) faces the object (10).

15. The object information acquiring apparatus according to claim 14, wherein
the information processing unit (15) is configured to acquire the characteristic information by using the electrical signal originating from an echo wave, which is the acoustic wave generated from the light absorber (8) disposed on the light-absorber supporting member (7) and reflected by the object (10), and the characteristic information by using the electrical signal originating from a photoacoustic wave generated from inside of the object (10) irradiated with the light.

**Patentansprüche**

1. Handgehaltene Sonde (1), mit:

einem Griffabschnitt (2);
einer Vielzahl von Erfassungselementen (4), die dazu eingerichtet sind, um eine akustische Welle zu empfangen und ein elektrisches Signal auszugeben;

einer Erfassungsfläche (3), auf der die Vielzahl von Erfassungselementen (4) angeordnet sind; und einem Lichtabsorber-Trägerelement (7), in dem ein Lichtabsorber (8) angeordnet ist, wobei der Lichtabsorber (8) das von einer Lichtquelle (13) emittierte Licht absorbiert und eine akustische Welle generiert, wobei wenn die handgehaltene Sonde (1) derart gegen ein Objekt (10) gedrückt wird, dass die Erfassungsfläche (3) dem Objekt (10) zugewandt ist, ist das Lichtabsorber-Trägerelement (7) zwischen dem Objekt (10) und der Erfassungsfläche (3) positioniert, in Kontakt mit dem Objekt (10), wobei, die Erfassungsfläche (3) dazu eingerichtet ist, um eine Form aufzuweisen, die relativ zum dem Objekt (10) konkav ist.

2. Handgehaltene Sonde nach Anspruch 1, ferner mit einem Bestrahlungsende (6a), das auf der Erfassungsfläche (3) angeordnet ist und das Licht emittiert.

3. Handgehaltene Sonde (1) nach Anspruch 1 oder 2, wobei
das Lichtabsorber-Trägerelement (7) eine Membran ist; und
der Lichtabsorber an der Membran bereitgestellt ist.

4. Handgehaltene Sonde (1) nach Anspruch 3, ferner mit einem Kopplungsmedium (9), das zwischen das Lichtabsorber-Trägerelement (7) und die Erfassungsfläche (3) gefüllt ist.

5. Handgehaltene Sonde (1) nach Anspruche 1 oder 2, wobei
das Lichtabsorber-Trägerelement (7) ein beutelförmiges Element ist; und
der Lichtabsorber an dem Beutel bereitgestellt ist.

6. Handgehaltene Sonde nach Anspruch 5, ferner mit einem Kopplungsmedium (9), das in das beutelförmige Element gefüllt ist.

7. Handgehaltene Sonde (1) nach einem der Ansprüche 3 bis 6, wobei der Lichtabsorber (8) punktförmig ist.

8. Handgehaltene Sonde (1) nach einem der Ansprüche 3 bis 7, mit einer Vielzahl von Lichtabsorbern, die dazu eingerichtet sind, um voneinander unterschiedliche Lichtabsorptionseigenschaften aufzuweisen.

9. Handgehaltene Sonde (1) nach Anspruch 2, wobei
das Lichtabsorber-Trägerelement (7) eine Membran ist, und
der Lichtabsorber (8) an der Membran auf einer optischen Achse (5) bereitgestellt ist, die sich vom Strahlungsende (6a) aus erstreckt.

10. Handgehaltene Sonde (1) nach Anspruch 1 oder 2, wobei
das Lichtabsorber-Trägerelement (7) ein Gel ist, und
der Lichtabsorber innerhalb des Gels bereitgestellt ist.

11. Handgehaltene Sonde (1) nach Anspruch 10, wobei der Lichtabsorber (8) flächenförmig ist.

12. Handgehaltene Sonde (1) nach Anspruch 11, wobei der flächenförmige Lichtabsorber (8) dazu eingerichtet ist, um einen Krümmungsradius aufzuweisen, der im Wesentlichen gleich dem der Erfassungsfläche (3) ist.

13. Handgehaltene Sonde (1) nach einem der Ansprüche 1 bis 12, wobei das Lichtabsorber-Trägerelement (7) lösbar an dem Griffabschnitt (2) befestigt ist.

14. Objektinformations-Erfassungsvorrichtung, mit:

der handgehaltenen Sonde (1) nach einem der Ansprüche 1 bis 13; und
einer Informationsverarbeitungseinheit (15), wobei
die Informationsverarbeitungseinheit (15) dazu eingerichtet ist, um charakteristische Informationen in Bezug auf das Innere eines Objekts unter Verwendung eines elektrischen Signals zu erfassen, das von einer akustischen Welle herrührt, die sich von dem Objekt (10) ausgebreitet hat, wobei die handgehaltene Sonde (1) derart gegen das Objekt (10) gedrückt wird, dass die Erfassungsfläche (3) dem Objekt (10) zugewandt ist.

15. Objektinformations-Erfassungsvorrichtung nach Anspruch 14, wobei

die Informationsverarbeitungseinheit (15) dazu eingerichtet ist, um die charakteristischen Informationen unter Verwendung des elektrischen Signals, das von einer Echowelle herrührt, die die akustische Welle ist, die von dem Lichtabsorber (8) generiert wird, der auf dem Lichtabsorber-Trägerelement (7) angeordnet ist, und von dem Objekt (10) reflektiert wird, und die charakteristischen Informationen unter Verwendung des elektrischen Signals, das von einer photoakustischen Welle herrührt, die vom Inneren des mit dem Licht bestrahlten Objekts (10) generiert wird, zu erlangen.

**Revendications**

1.  Sonde de type à main (1), comprenant :

    une partie de préhension (2) ;
    une pluralité d'éléments de détection (4) configurés pour recevoir une onde acoustique et pour délivrer en sortie un signal électrique ;
    une surface de détection (3) au niveau de laquelle sont disposés les éléments de la pluralité d'éléments de détection (4) ; et
    un élément de support (7) d'absorbeur de lumière au niveau duquel est disposé un absorbeur de lumière (8), l'absorbeur de lumière (8) absorbant de la lumière émise à partir d'une source de lumière (13) et générant une onde acoustique, où
    lorsque la sonde de type à main (1) est appuyée contre un objet (10) de sorte que la surface de détection (3) fasse face à l'objet (10), l'élément de support (7) d'absorbeur de lumière est disposé entre l'objet (10) et la surface de détection (3) en contact avec l'objet, où
    la surface de détection (3) est configurée pour avoir une forme concave par rapport à l'objet (10).

2.  Sonde de type à main (1) selon la revendication 1, comprenant en outre une extrémité d'exposition à un rayonnement (6a) disposée sur la surface de détection (3) et émettant la lumière.

3.  Sonde de type à main (1) selon l'une quelconque de la revendication 1 ou 2, dans laquelle
    l'élément de support (7) d'absorbeur de lumière est une membrane ; et
    l'absorbeur de lumière est disposé au niveau de la membrane.

4.  Sonde de type à main (1) selon la revendication 3, comprenant en outre un milieu de couplage (9) remplissant l'espace entre l'élément de support (7) d'absorbeur de lumière et la surface de détection (3).

5.  Sonde de type à main (1) selon l'une quelconque de la revendication 1 ou 2, dans laquelle
    l'élément de support (7) d'absorbeur de lumière est un élément en forme de sac ; et
    l'absorbeur de lumière est disposé au niveau du sac.

6.  Sonde de type à main (1) selon la revendication 5, comprenant en outre un milieu de couplage (9) remplissant l'élément en forme de sac.

7.  Sonde de type à main (1) selon l'une quelconque des revendications 3 à 6, dans laquelle l'absorbeur de lumière (8) est de type point.

8.  Sonde de type à main (1) selon l'une quelconque des revendications 3 à 7, comprenant une pluralité d'absorbeurs de lumière configurés pour avoir des caractéristiques d'absorption de lumière mutuellement différentes.

9.  Sonde de type à main (1) selon la revendication 2, dans laquelle
    l'élément de support (7) d'absorbeur de lumière est une membrane, et
    l'absorbeur de lumière (8) est disposé au niveau de la membrane sur un axe optique (5) qui s'étend à partir de l'extrémité d'exposition à un rayonnement (6a).

10. Sonde de type à main (1) selon l'une quelconque de la revendication 1 ou 2, dans laquelle
    l'élément de support (7) d'absorbeur de lumière est un gel, et
    l'absorbeur de lumière est disposé à l'intérieur du gel.

11. Sonde de type à main (1) selon la revendication 10, dans laquelle l'absorbeur de lumière (8) est de type feuille.

**12.** Sonde de type à main (1) selon la revendication 11, dans laquelle l'absorbeur de lumière de type feuille (8) est configuré pour avoir un rayon de courbure sensiblement égal à celui de la surface de détection (3).

**13.** Sonde de type à main (1) selon l'une quelconque des revendications 1 à 12, dans laquelle l'élément de support (7) d'absorbeur de lumière est fixé amovible à la partie de préhension (2).

**14.** Appareil d'acquisition d'informations d'objet, comprenant :

la sonde de type à main (1) selon l'une quelconque des revendications 1 à 13 ; et
une unité de traitement d'informations (15), où
l'unité de traitement d'informations (15) est configurée pour acquérir des informations caractéristiques se rapportant à l'intérieur d'un objet (10) au moyen d'un signal électrique ayant pour origine une onde acoustique qui s'est propagée à partir de l'objet (10), la sonde de type à main (1) étant appuyée contre l'objet (10) de sorte que la surface de détection (3) fasse face à l'objet (10).

**15.** Appareil d'acquisition d'informations d'objet selon la revendication 14, dans lequel
l'unité de traitement d'informations (15) est configurée pour acquérir les informations caractéristiques au moyen du signal électrique ayant pour origine une onde d'écho, qui est l'onde acoustique générée à partir de l'absorbeur de lumière (8) disposé sur l'élément de support (7) d'absorbeur de lumière et réfléchie par l'objet (10), et les informations caractéristiques au moyen du signal électrique ayant pour origine une onde photo-acoustique générée à partir de l'intérieur de l'objet (10) exposé à un rayonnement de la lumière.

FIG. 1

FIG. 2

**FIG. 3A**

START → MEASUREMENT PREPARATION (S301) → LIGHT IRRADIATION (S302) → ACOUSTIC WAVE RECEPTION (S303) → SIGNAL PROCESSING (S304) → IMAGE RECONSTRUCTION (S305) → IMAGE DISPLAY (S306) → END

**FIG. 3B**

S301 → EMIT LIGHT FROM LIGHT SOURCE (S3021) → PHOTOACOUSTIC WAVE GENERATED FROM LIGHT ABSORBERS IN OBJECT AND ON LIGHT-ABSORBER SUPPORTING MEMBER (S3022) → S303

**FIG. 3C**

S304 → RECONSTRUCT PHOTOACOUSTIC IMAGE (S3051) → DETECT ABSORBER POSITION (S3052) → RECONSTRUCT ULTRASONIC IMAGE (S3053) → DETECT ABSORBER POSITION (S3054) → CREATE COMBINED IMAGE OF PHOTOACOUSTIC IMAGE AND ULTRASONIC IMAGE (S3055) → S306

FIG. 4

FIG. 5

FIG. 6A                    FIG. 6B

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012090742 A1

- JP 2013233238 A

**Non-patent literature cited in the description**

- **J. J. NIEDERHAUSER ; M. JAEGER ; R. LEMOR ; P. WEBER ; M. FRENZ.** Combined ultrasound and optoacoustic system for real-time high-contrast vascular imaging in vivo. *IEEE Trans. Med. Imaging,* 2005, vol. 24, 436-440